# EUROPEAN PATENT SPECIFICATION

(11) **EP 2 103 278 B1**
(45) Date of publication and mention of the grant of the patent: **05.09.2012**
(21) Application number: 09153745.6
(22) Date of filing: 26.02.2009
(51) Int. Cl.: A61F 2/06

(54) **Stent-graft or graft with durability or stiffness enhancing elements**
Gefässprothese mit Verbesserungselementen für erhöhte Lebensdauer und Flexibilität
Prothèse vasculaire avec éléments de renfort de la rigidité ou de la durabilité

(30) Priority: 17.03.2008 US 49445
(43) Date of publication of application: 23.09.2009
(73) Proprietor: Cordis Corporation, Miami Lakes, FL 33014 (US)
(72) Inventor: Fleming, James, Bethlehem Pennsylvania, PA 18020 (US); Majercak, David C., Stewartsville New Jersey, NJ 08886 (US); Park, Jin S., Parsippany New Jersey, NJ 07054 (US)
(74) Representative: Brown, George Laurence

(56) References cited:
- EP-A- 1 621 159
- EP-A- 1 839 624
- US-A1- 2002 077 634
- US-A1- 2007 270 942

## Description

The present invention relates to aneurismal repair devices, and more particularly, to materials for improving the wear resistance of grafts and stent-grafts.

An aneurysm is an abnormal dilation of a layer or layers of an arterial wall, usually caused by a systemic collagen synthetic or structural defect. An abdominal aortic aneurysm is an aneurysm in the abdominal portion of the aorta, usually located in or near one or both of the two iliac arteries or near the renal arteries. The aneurysm often arises in the infrarenal portion of the diseased aorta, for example, below the kidneys. A thoracic aortic aneurysm is an aneurysm in the thoracic portion of the aorta. When left untreated, the aneurysm may rupture, usually causing rapid fatal hemorrhaging.

Aneurysms may be classified or typed by their position as well as by the number of aneurysms in a cluster. Typically, abdominal aortic aneurysms may be classified into five types. A Type I aneurysm is a single dilation located between the renal arteries and the iliac arteries. Typically, in a Type I aneurysm, the aorta is healthy between the renal arteries and the aneurysm and between the aneurysm and the iliac arteries.

A Type II A aneurysm is a single dilation located between the renal arteries and the iliac arteries. In a Type II A aneurysm, the aorta is healthy between the renal arteries and the aneurysm, but not healthy between the aneurysm and the iliac arteries. In other words, the dilation extends to the aortic bifurcation. A Type II B aneurysm comprises three dilations. One dilation is located between the renal arteries and the iliac arteries. Like a Type II A aneurysm, the aorta is healthy between the aneurysm and the renal arteries, but not healthy between the aneurysm and the iliac arteries. The other two dilations are located in the iliac arteries between the aortic bifurcation and the bifurcations between the external iliacs and the internal iliacs. The iliac arteries are healthy between the iliac bifurcation and the aneurysms. A Type II C aneurysm also comprises three dilations. However, in a Type II C aneurysm, the dilations in the iliac arteries extend to the iliac bifurcation.

A Type III aneurysm is a single dilation located between the renal arteries and the iliac arteries. In a Type III aneurysm, the aorta is not healthy between the renal arteries and the aneurysm. In other words, the dilation extends to the renal arteries.

A ruptured abdominal aortic aneurysm is presently the thirteenth leading cause of death in the United States. The routine management of abdominal aortic aneurysms has been surgical bypass, with the placement of a graft in the involved or dilated segment. Although resection with a synthetic graft via a transperitoneal or retroperitoneal procedure has been the standard treatment, it is associated with significant risk. For example, complications include perioperative myocardial ischemia, renal failure, erectile impotence, intestinal ischemia, infection, lower limb ischemia, spinal cord injury with paralysis, aorta-enteric fistula, and death. Surgical treatment of abdominal aortic aneurysms is associated with an overall mortality rate of five percent in asymptomatic patients, sixteen to nineteen percent in symptomatic patients, and is as high as fifty percent in patients with ruptured abdominal aortic aneurysms.

Disadvantages associated with conventional surgery, in addition to the high mortality rate, include an extended recovery period associated with the large surgical incision and the opening of the abdominal cavity, difficulties in suturing the graft to the aorta, the loss of the existing thrombosis to support and reinforce the graft, the unsuitability of the surgery for many patients having abdominal aortic aneurysms, and the problems associated with performing the surgery on an emergency basis after the aneurysm has ruptured. Further, the typical recovery period is from one to two weeks in the hospital and a convalescence period, at home, ranging from two to three months or more, if complications ensue. Since many patients having abdominal aortic aneurysms have other chronic illnesses, such as heart, lung, liver and/or kidney disease, coupled with the fact that many of these patients are older, they are less than ideal candidates for surgery.

The occurrence of aneurysms is not confined to the abdominal region. While abdominal aortic aneurysms are generally the most common, aneurysms in other regions of the aorta or one of its branches are possible. For example, aneurysms may occur in the thoracic aorta. As is the case with abdominal aortic aneurysms, the widely accepted approach to treating an aneurysm in the thoracic aorta is surgical repair, involving replacing the aneurysmal segment with a prosthetic device. This surgery, as described above, is a major undertaking, with associated high risks and with significant mortality and morbidity.

Over the past five years, there has been a great deal of research directed at developing less invasive, endovascular, i.e., catheter directed, techniques for the treatment of aneurysms, specifically abdominal aortic aneurysms. This has been facilitated by the development of vascular stents, which can and have been used in conjunction with standard or thin-wall graft material in order to create a stent-graft or endograft. The potential advantages of less invasive treatments have included reduced surgical morbidity and mortality along with shorter hospital and intensive care unit stays.

Stent-grafts or endoprostheses are now Food and Drug Administration (FDA) approved and commercially available. Their delivery procedure typically involves advanced angiographic techniques performed through vascular accesses gained via surgical cut down of a remote artery, which may include the common femoral or brachial arteries. Over a guidewire, the appropriate size introducer will be placed. The catheter and guidewire are passed through the aneurysm. Through the introducer, the stent-graft will be advanced to the appropriate position. Typical deployment of the stent-graft device requires withdrawal of an outer sheath while maintaining the position of the stent-graft with an inner-stabilizing device. Most stent-grafts are self-expanding; however, an additional angioplasty procedure, e.g., balloon angioplasty, may be required to secure the position of the stent-graft. Following the placement of the stent-graft, standard angiographic views may be obtained.

Due to the large diameter of the above-described devices, typically greater than twenty French (3F=1 mm), arteriotomy closure typically requires open surgical repair. Some procedures may require additional surgical techniques, such as hypogastric artery embolization, vessel ligation, or surgical bypass in order to adequately treat the aneurysm or to maintain blood flow to both lower extremities. Likewise, some procedures will require additional advanced catheter directed techniques, such as angioplasty, stent placement and embolization, in order to successfully exclude the aneurysm and efficiently manage leaks.

While the above-described endoprostheses represent a significant improvement over conventional surgical techniques, there is a need to improve the endoprostheses, their method of use and their applicability to varied biological conditions. Accordingly, in order to provide a safe and effective alternate means for treating aneurysms, including abdominal aortic aneurysms and thoracic aortic aneurysms, a number of difficulties associated with currently known endoprostheses and their delivery systems must be overcome. One concern with the use of endoprostheses is the prevention of endo-leaks and the disruption of the normal fluid dynamics of the vasculature. Devices using any technology should preferably be simple to position and reposition as necessary, should preferably provide an acute, fluid tight seal, and should preferably be anchored to prevent migration without interfering with normal blood flow in both the aneurysmal vessel as well as branching vessels. In addition, devices using the technology should preferably be able to be anchored, sealed, and maintained in bifurcated vessels, tortuous vessels, highly angulated vessels, partially diseased vessels, calcified vessels, odd shaped vessels, short vessels, and long vessels. In order to accomplish this, the endoprostheses should preferably be highly durable, extendable and re-configurable while maintaining acute and long-term fluid tight seals and anchoring positions.

The endoprostheses should also preferably be able to be delivered percutaneously utilizing catheters, guidewires and other devices which substantially eliminate the need for open surgical intervention. Accordingly, the diameter of the endoprostheses in the catheter is an important factor. This is especially true for aneurysms in the larger vessels, such as the thoracic aorta. In addition, the endoprostheses should preferably be percutaneously delivered and deployed such that surgical cut down is unnecessary.

Many aneurismal repair devices utilize a woven graft material in combination with a supporting intraluminal scaffold or stent. Fiber wear and fiber separation within the graft material of the components comprising the aneurismal repair devices may be a potential problem. The pulsatile movement of the artery in which the repair device is positioned, for example, the aorta, causes the stent to rub against the graft material thereby potentially resulting in holes that may cause endoleaks. Another potential problem caused by the interaction of the stent structures and the graft material is the separation of the graft fibers. This is caused when part of the stent structure is pressed into the graft material and forces adjacent fibers to separate. This condition may be triggered during manufacturing while handling and loading the device, or in vivo, in cases of extreme bending or movement. Accordingly, it would be highly advantageous to develop a means for preventing fiber wear and fiber separation.

US-A-2007/0270942 relates to stent grafts with structural scaffolds that undergo controlled galvanic corrosion in situ. A galvanic cell is affixed to the vessel luminal wall-contacting surface using a biocompatible cyanoacrylate adhesive.

The present invention overcomes the disadvantages associated with currently utilized aneurismal repair devices due to fiber wear and fiber separation within the components of the system as briefly described above.

In accordance with one aspect, the present invention is directed to an aneurismal repair system as defined in appended claim 1 which comprises an epoxy affixed to predetermined areas of the graft structure. In one embodiment an aneurismal repair system comprises at least one stent segment, graft material affixed, via attachment elements, to the at least one stent segment to form an endoprosthesis, and the epoxy is affixed to the graft structure proximate an area of fixed contact between the at least one stent segment and the graft structure to enhance durability.

In accordance with another embodiment, the aneurismal repair system comprises at least one stent segment, graft material affixed, via attachment elements, to the at least one stent segment to form an endoprosthesis, and the epoxy is affixed to a predetermined area of the graft material to enhance stiffness.

Many aneurismal repair devices utilize a woven graft material in combination with a supporting intraluminal scaffold or stent. Fiber wear and fiber separation within the graft material of the components comprising the aneurismal repair devices may be a potential problem. The pulsatile movement of the artery in which the repair device is positioned, for example, the aorta, causes the stents to rub against the graft material thereby potentially resulting in holes that may cause endoleaks. Another potential problem caused by the interaction of the stent structures and the graft material is the separation of the graft fibers. This is caused when part of the stent structure is pressed into the graft material and forces adjacent fibers to separate. This condition may be triggered during manufacturing while handling and loading the device or in vivo in cases of extreme bending or movement.

Numerous modification to the type of weave used in a graft or the design of the stent structures have been made over the years to prevent graft wear and separation. Many of these methods are complicated and expensive to incorporate. The present invention is directed to adding an epoxy material to the graft material in areas of expected interaction with the stent structure. The addition of the epoxy should preferably prevent the fibers from separating and also improve wear resistance in two ways. Initially, the epoxy will provide additional material in between the graft fibers, which will delay the wear process and if a graft fiber eventually does break due to wear, the break will be isolated from the rest of the graft material by the epoxy. This will prevent propagation of the hole and weakening of the graft adjacent to the tear.

This simple solution addresses may potential problems encountered by any medical implant utilizing a woven graft as part of the structure. In addition, this simple solution is inexpensive and does not require complicated manufacturing steps or a highly skilled operator to apply in manufacturing.

Embodiments of the invention will now be described by way of example with reference to the accompanying drawings, in which:
Figure 1 is a diagrammatic representation of the exemplary anchoring and sealing prosthesis in accordance with the present invention.
Figure 2 is a diagrammatic representation of an exemplary anchoring and sealing prosthesis with no graft material and/or stitching in certain locations in accordance with the present invention.
Figure 3 is an elevational view of an endovascular graft in accordance with the present invention.
Figure 4 is a perspective view of an expanded stent segment of the endovascular graft in accordance with the present invention.
Figure 4A is a fragmentary perspective view of a portion of the stent segment of Figure 4.
Figure 4B is a fragmentary perspective view of a portion of the stent segment of Figure 4.
Figure 4C is an enlarged plan view of a section of the stent segment of Figure 4.
Figure 4D is an enlarged plan view of a section of the stent segment of Figure 4.
Figure 5 is a perspective view of another expanded stent segment of the endovascular graft in accordance with the present invention.
Figure 6 is an elevational view of an endovascular graft in accordance with the present invention.
Figure 7 is a diagrammatic representation of a reinforced section of graft material in accordance with the present invention.
Figure 8 is a diagrammatic representation of a reinforced suture line in accordance with the present invention.

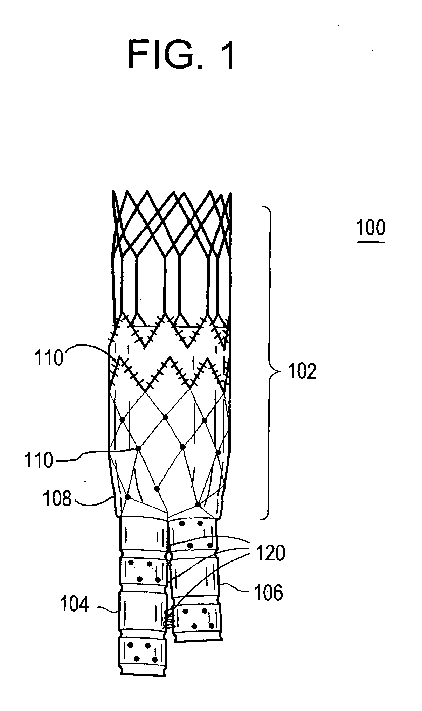

Referring to Figure 1, there is illustrated an exemplary embodiment of an anchoring and sealing component 100 of an aneurysm repair system. The anchoring and sealing component 100 comprises a trunk section 102 and a bifurcated section, including two legs 104, 106. Graft material 108, described in detail below, is affixed to at least a portion of the trunk section 102 and to all of the legs 104, 106. The graft material may be attached via any number of means. In the exemplary embodiment, the graft material 108 is attached to various portions of the underlying structure by sutures 110. As illustrated, the graft material 108 is affixed with a continuous stitch pattern on the end of the trunk section 102 and by single stitches elsewhere. It is important to note that any stitch pattern may be utilized, and other devices, such as staples, may be utilized to connect the graft material 108 to the underlying structure. The sutures 110 may comprise any suitable biocompatible material that is preferably highly durable and wear resistant.

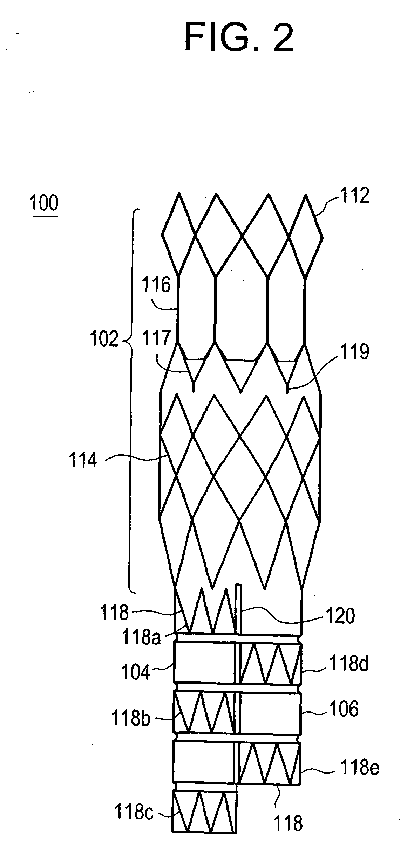

The underlying structure of the trunk section 102, as illustrated in Figure 2, comprises a substantially tubular stent structure or lattice comprising multiple stent sections. The stent or lattice structure comprises a single row of substantially diamond shaped elements 112 on one end, multiple rows of substantially diamond shaped elements 114 on the other end, a plurality of longitudinal struts 116 and a single, substantially zigzag shaped stent element 117. The plurality of longitudinal struts 116 are connected to the apexes of the substantially diamond shaped elements 114. The single, substantially zigzag shaped stent element 117 comprises a number of barbs 119 protruding therefrom for anchoring the device in the vessel to be repaired. This exemplary embodiment may be utilized for anchoring and sealing in positions wherein there are branches off the main artery. For example, this exemplary embodiment may be utilized for supra-renal anchoring. Accordingly, the graft material 108 is only attached below the longitudinal struts 116 so that blood may flow into the renal arteries from the aorta. Infra-renal designs are also possible.

The underlying structure of the bifurcated section, as illustrated in Figure 2, comprises a plurality of individual, substantially tubular stent elements 118. Each stent element 118 comprises a substantially zigzag pattern. As illustrated, leg 104 comprises three stent elements 118a, 118b, 118c and leg 106 comprises two stent elements 118d, 118e. As illustrated, in this exemplary embodiment, the stent elements do not line up and the legs are of two different lengths. This exemplary design allows for nesting of the legs 104, 106 such that the profile of the device is reduced.

In order to compensate for the missing stent elements, the legs are connected at the bifurcation as illustrated in Figure 1. The legs 104, 106 may be connected in any suitable manner. In the exemplary embodiment, the two legs 104, 106 are connected by suturing them together. The sutures 120 connect the graft material 108 on each leg 104, 106 together. The sutures may be non-biodegradable or biodegradable. Biodegradable sutures would dissolve over time thereby allowing the two legs to move independently.

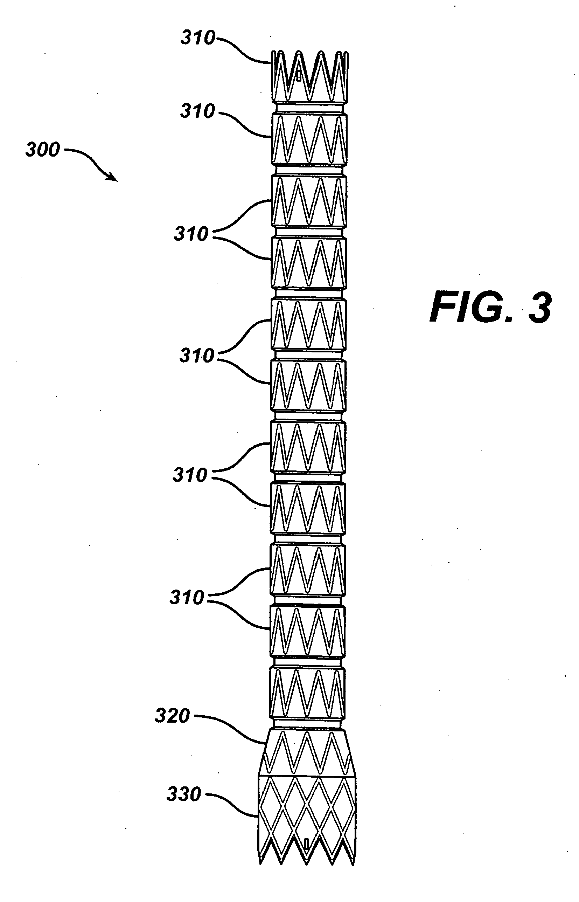

Referring now to Figure 3, there is illustrated an exemplary embodiment of an endovascular graft 300 of an aneurysm repair system. The exemplary endovascular graft 300 comprises one or more first stent segments 310, one second stent segment 320 and a third stent segment 330. In a typical use scenario, the third stent segment 330 would be anchored in healthy tissue below the aneurysm and the uppermost first stent segment 310 would be in fluid communication with the anchoring and sealing component 100. The second stent segment 320 comprises a tapered profile, having a diameter at one end equal to that of the first stent segment 310 and a diameter at the other end equal to that of the third stent segment 330. The length of the endovascular graft 300 may be adjusted by varying the number of first stent segments 310 utilized.

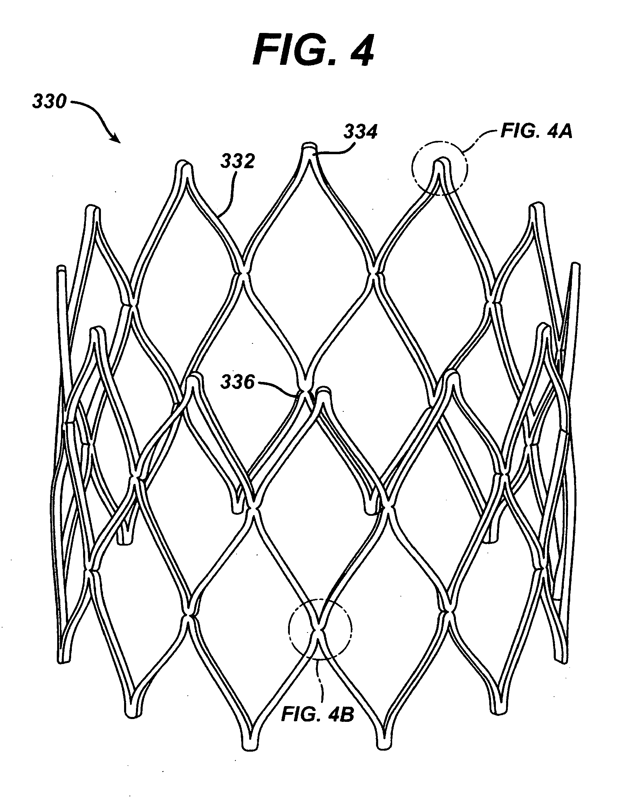

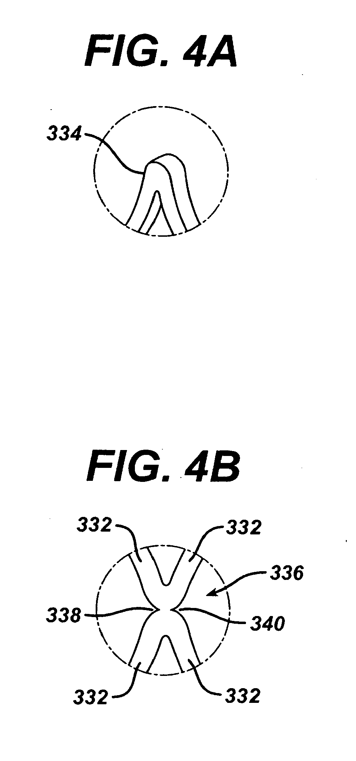

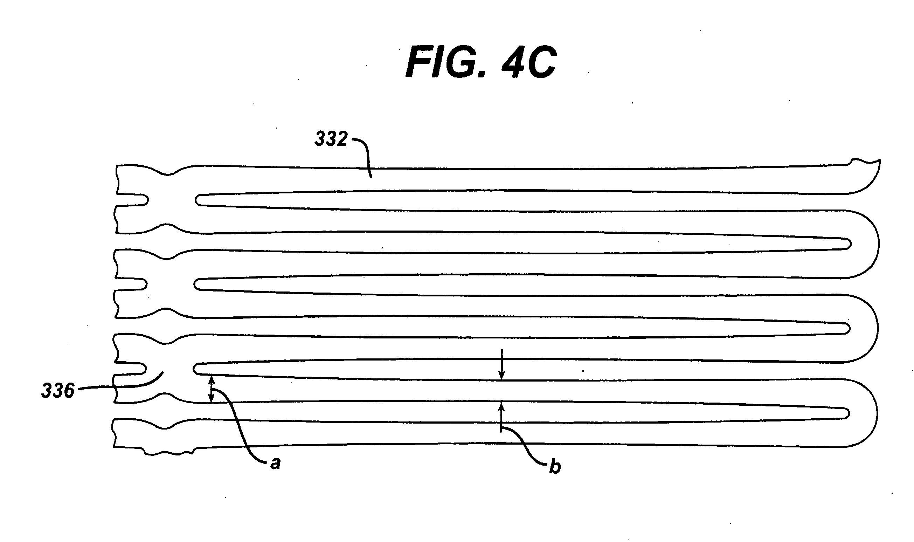

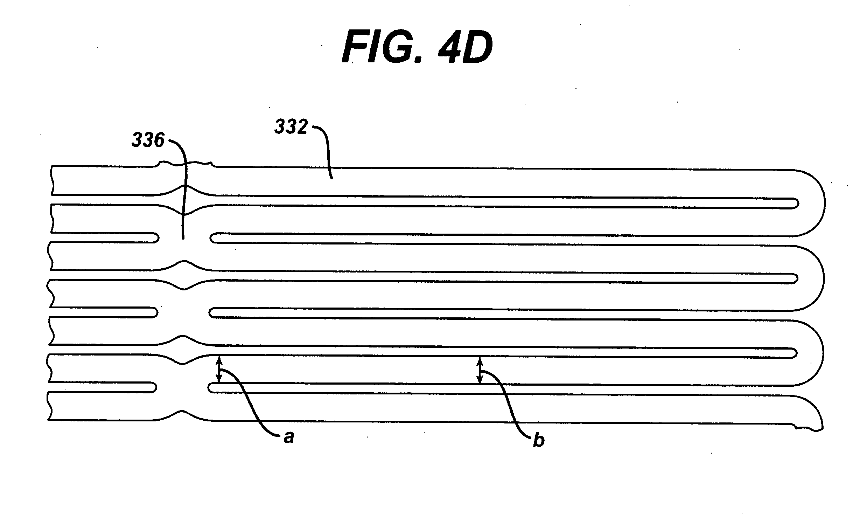

Figure 4 is a detailed perspective view of an exemplary embodiment of the third stent segment 330. The third stent segment 330 comprises a plurality of struts 332 connected in a substantially zigzag pattern. As illustrated, the exemplary third stent segment 330 comprises three sets of zigzag-connected struts 332, thereby forming substantially diamond-shaped cells. The non- connected apex 334 of each diamond shaped cell, illustrated in greater detail in Figure 4A, comprises a smooth, uniform width curved region formed at the intersection of two struts 332 of each diamond-shaped cell. This shape is cut directly into the stent segment 330 during the initial machining steps, typically laser cutting, and is maintained during all subsequent finishing processing. The junctions 336 between the zigzag- connected struts 332, illustrated in greater detail in Figure 4B occurs at the intersection of four struts 332. Preferably, each junction 336 of four struts 332 comprises two indentations 338 and 340 as illustrated in Figure 4B.

The regions proximate the non-connected apexes 334 and the junctions 336 are generally the highest stress regions in the third stent segment 330. To minimize the stresses in these regions, these regions are designed to maintain uniform beam widths proximate where the struts 332 interconnect. Beam width refers to the width of a strut junction 336. Indentations 338 and 340 are cut or machined into the junctions 336 to maintain a uniform beam width in this area, which is generally subject to the highest stress. Essentially, by designing the junctions 336 to maintain uniform beam widths, the stress and strain that would normally build up in a concentrated area, proximate the junction 336, is allowed to spread out into the connecting regions, thereby lowering the peak values of the stress and strain in the stent structure.

To further minimize the maximum stresses in the struts 332 of the third stent segment 330, the struts 332 may have a tapering width. For example, in one exemplary embodiment, the struts 332 may be designed to become wider as it approaches a junction 336. Figure 4C is an enlarged partial view of the third sent segment 330 in its expanded conditions which illustrates the tapering width of the struts 332. In this exemplary embodiment, the strut 332 proximate the junction 336 (width a) is about 0.025 cm and gradually tapers to a dimension of about 0.0178 cm in the mid-region of the strut 332 (width b). By tapering the struts' widths, the stresses in the struts 332 adjacent the junction 336 is spread out away from the junction 336. The tapering of the struts 332 is accomplished during the machining of the tube of material from which the stent 330 is cut. However, by tapering the struts 332 in this manner, there is a tradeoff. The stent segment 330 becomes somewhat less resistant to localized deformations, caused for example, by a protrusion within the vessel lumen. This localized deformation may lead to a local torsional loading on some of the struts 332, and, therefore, since the struts 332 in this exemplary embodiment have a relatively significant portion of their length with a reduced width, their torsional rigidity is reduced.

If maximizing the resistance to localized deformation is preferred, the struts 332 may be maintained at a uniform width, or more preferably have a reverse taper, as illustrated in Figure 4D, wherein the width at point a is less than the width at point b. In this exemplary embodiment, the reverse taper struts 332 are about 0.025 cm proximate the junction 336 and about 0.028 cm in the central region of the struts. While this reverse taper tends to increase the stresses somewhat proximate the junctions 336, this increase is very small relative to the decrease in stresses gained by having the side indentations 338, 340 illustrated in Figure 4B, as well as the uniform width connections illustrated in Figure 4A. In addition, since the reverse taper serves to increase the torsional rigidity of the strut 332, the stent structure resists local deformation and tends to maintain a substantially circular cross-sectional geometry, even if the lumen into which the stent is positioned in non-circular in cross-section.

In a preferred exemplary embodiment, the third stent segment 330 is fabricated from a laser cut tube, of initial dimensions 0.229 cm inside diameter by 0.318 cm outside diameter. The struts 332 are preferably 0.0229 cm wide adjacent the four strut junctions 336 and six mm long, with a reverse taper strut width. Also, to minimize the number of different diameter combination of grafts systems, it is preferred that the third stent segment 330 have an expanded diameter of sixteen mm. Similarly, the proximal portion of the graft material forming the legs is flared, having a diameter of sixteen mm. This single diameter for the third stent segment of the graft system would enable its use in arteries having a non-aneurysmal region of a diameter from between eight and fourteen mm in diameter. It is also contemplated that multiple diameter combinations of third stent segment 330 and graft flare would be desirable.

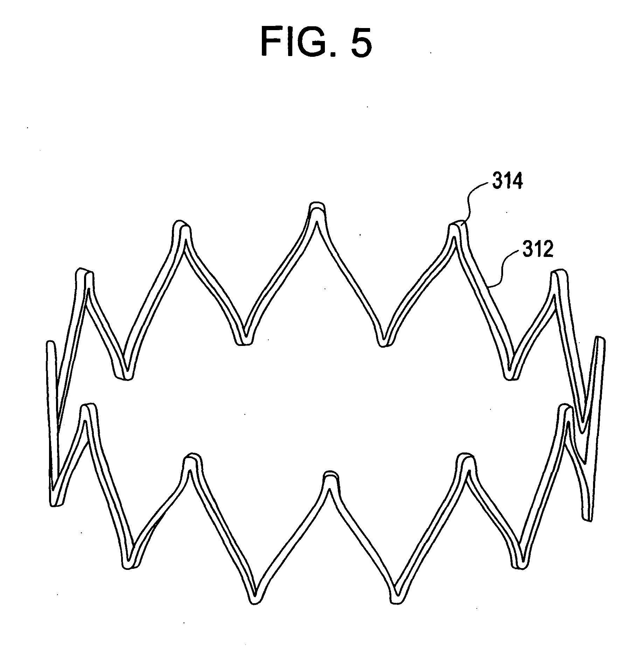

Referring back to Figure 3, the one or more first stent segments 310 are also formed from a shape set laser cut tube, similar to the third stent segment 330 described above. The one or more first stent segments 310 comprise a single circumferential row of zigzag or sinusoidally arranged elements. In the exemplary embodiment illustrated in Figure 3, and in greater detail in Figure 5, the first stent segment 310 comprises ten zigzag or sinusoidal undulations. The one or more first stent segments 310 are formed with uniform width connections at the intersections 314 of the struts 312 forming the zigzag or sinusoidal pattern. The one or more first stent segments 310 are preferably cut from tubing having an inside diameter of 0.251 cm and an outside diameter of 0.317 cm. The strut widths are preferably about 0.33 cm wide adjacent strut intersections 314 and the struts 312 are preferably seven mm long and the one or more first stent segments 310 are preferably eleven mm in diameter when expanded.

The second stent segment 320 comprises a tapered profile, having a diameter at one end which is the same as the one or more first stent segments 310, and a diameter at the other end matching the diameter of the third stent segment 330. The second stent segment 320 is identical to the one or more first stent segments 310 except for the taper.

As is explained in detail subsequently, the stent segments 310, 320 and 330 are secured in position by the graft material.

Nitinol is utilized in a wide variety of applications, including medical device applications as described herein. Nitinol or Ni-Ti alloys are widely utilized in the fabrication or construction of medical devices for a number of reasons, including its biomechanical compatibility, its biocompatibility, its fatigue resistance, its kink resistance, its uniform plastic deformation, its magnetic resonance imaging compatibility, its constant and gentle outward pressure, its dynamic interference, its thermal deployment capability, its elastic deployment capability, its hysteresis characteristics and because it is modestly radiopaque.

Nitinol, as described above, exhibits shape memory and/or superelastic characteristics. Shape memory characteristics may be simplistically described as follows. A metallic structure, for example a Nitinol tube that is in an Austenite phase may be cooled to a temperature such that it is in the Martensite phase. Once in the Martensite, the Nitinol tube may be deformed into a particular configuration or shape by the application of stress. As long as the Nitinol tube is maintained in the Martensite phase, the Nitinol tube will remain in its deformed shape. If the Nitinol tube is heated to a temperature sufficient to cause the Nitinol tube to reach the Austenite phase, the Nitinol tube will return to its original or programmed shape. The original shape is programmed to be a particular shape by well known techniques. Superelastic characteristics may be simplistically described as follows. A metallic structure, for example, a Nitinol tube that is in an Austenite phase may be deformed to a particular shape or configuration by the application of mechanical energy. The application of mechanical energy causes a stress induced Martensite phase transformation. In other words, the mechanical energy causes the Nitinol tube to transform from the Austenite phase to the Martensite phase. By utilizing the appropriate measuring instruments, one can determine that the stress from the mechanical energy causes a temperature drop in the Nitinol tube. Once the mechanical energy or stress is released, the Nitinol tube undergoes another mechanical phase transformation back to the Austenite phase and thus its original or programmed shape. As described above, the original shape is programmed by well known techniques. The Martensite and Austenite phases are common phases in many metals.

Medical devices constructed from Nitinol are typically utilized in both the Martensite phase and/or the Austenite phase. The Martensite phase is the low temperature phase. A material in the Martensite phase is typically very soft and malleable. These properties make it easier to shape or configure the Nitinol into complicated or complex structures. The Austenite phase is the high temperature phase. A material in the Austenite phase is generally much stronger than the material in the Martensite phase. Typically, many medical devices are cooled to the Martensite phase for manipulation and loading into delivery systems, as described above with respect to stents and then when the device is deployed at body temperature, they return to the Austenite phase.

The first, second and third stent segments 310, 320, 330 are preferably self-expandable and formed from a shape memory alloy. Such an alloy may be deformed from an original, heat-stable configuration to a second, heat-unstable configuration. The application of a desired temperature causes the alloy to revert to an original heat-stable configuration. A particularly preferred shape memory alloy for this application is binary nickel titanium alloy comprising about 55.8 percent Ni by weight, commercially available under the trade designation NITINOL. This NiTi alloy undergoes a phase transformation at physiological temperatures. A stent made of this material is deformable when chilled. Thus, at low temperatures, for example, below twenty degrees centigrade, the stent is compressed so that it can be delivered to the desired location. The stent may be kept at low temperatures by circulating chilled saline solutions. The stent expands when the chilled saline is removed and it is exposed to higher temperatures within the patient's body, generally around thirty-seven degrees centigrade.

In preferred embodiments, each stent is fabricated from a single piece of alloy tubing. The tubing is laser cut, shape-set by placing the tubing on a mandrel, and heat-set to its desired expanded shape and size.

In preferred embodiments, the shape setting is performed in stages at five hundred degrees centigrade. That is, the stents are placed on sequentially larger mandrels and briefly heated to five hundred degrees centigrade. To minimize grain growth, the total time of exposure to a temperature of five hundred degrees centigrade is limited to five minutes. The stents are given their final shape set for four minutes at five hundred fifty degrees centigrade, and then aged to a temperature of four hundred seventy degrees centigrade to import the proper martensite to austenite transformation temperature, then blasted, as described in detail subsequently, before electropolishing. This heat treatment process provides for a stent that has a martensite to austenite transformation which occurs over a relatively narrow temperature range; for example, around fifteen degrees centigrade.

To improve the mechanical integrity of the stent, the rough edges left by the laser cutting are removed by combination of mechanical grit blasting and electropolishing. The grit blasting is performed to remove the brittle recast layer left by the laser cutting process. This layer is not readily removable by the electropolishing process, and if left intact, could lead to a brittle fracture of the stent struts. A solution of seventy percent methanol and thirty percent nitric acid at a temperature of minus forty degrees centigrade or less has been shown to work effectively as an electropolishing solution. Electrical parameters of the electropolishing are selected to remove approximately 0.00127 cm of material from the surfaces of the struts. The clean, electropolished surface is the final desired surface for attachment to the graft materials. This surface has been found to import good corrosion resistance, fatigue resistance, and wear resistance.

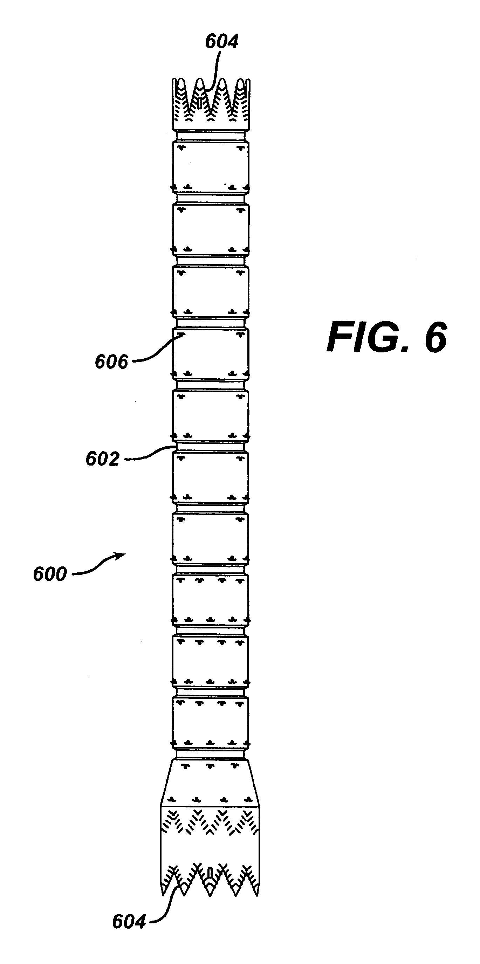

The graft material or component 600, as illustrated in Figure 6, may be made from any number of suitable biocompatible materials, including woven, knitted, sutured, extruded, or cast materials comprising polyester, polytetrafluoroethylene, silicones, urethanes, and ultralight weight polyethylene, such as that commercially available under the trade designation SPECTRA™. The materials may be porous or nonporous. Exemplary materials include a woven polyester fabric made from DACRON™ or other suitable PET-type polymers.

In one exemplary embodiment, the fabric for the graft material is a forty denier (denier is defined in grams of nine thousand meters of a filament or yarn), twenty-seven filament polyester yarn, having about seventy to one-hundred end yarns per cm per face and thirty-two to forty-six pick yarns per cm face. At this weave density, the graft material is relatively impermeable to blood flow through the wall, but is relatively thin, ranging between 0.08 and 0.12 mm in wall thickness.

The graft component 600 is a single lumen tube and preferably has a taper and flared portion woven directly from the loom, as illustrated for the endovascular graft 300 shown in Figure 3.

Prior to attachment of the graft component 600 to the stents 310, 320, 330, crimps are formed between the stent positions by placing the graft material on a shaped mandrel and thermally forming indentations in the surface. In the exemplary embodiment illustrated in Figures 3 and 6, the crimps 602 in the graft 400 are about two mm long and 0.5 mm deep. With these dimensions, the endovascular graft 300 can bend and flex while maintaining an open lumen. Also, prior to attachment of the graft component 600 to the stents 310, 320 330, the graft material is cut in a shape to mate with the end of each end stent.

As stated above, each of the stent segments 310, 320 and 330 is attached to the graft material 600. The graft material 600 may be attached to the stent segments 310, 320, 330 in any number of suitable ways. In one exemplary embodiment, the graft material 600 may be attached to the stent segments 310, 320, 330 by sutures.

The method of suturing stents in place is important for minimizing the relative motion or rubbing between the stent struts and the graft material. Because of the pulsatile motion of the vasculature and therefore the graft system, it is possible for relative motion to occur, particularly in areas where the graft system is in a bend, or if there are residual folds in the graft material, due to being constrained by the aorta or iliac arteries.

Ideally, each strut of each stent segment is secured to the graft material by sutures. In an exemplary embodiment, the suture material is blanket stitched to the stent segments at numerous points to securely fasten the graft material to the stent segments. As stated above, a secure hold is desirable in preventing relative motion in an environment in which the graft system experiences dynamic motion arising from pulsatile blood pressure, in addition to pulsation of the arteries that are in direct mechanical contact with the graft system. The stents nearest the aortic and iliac ends of the graft system (the uppermost first stent segment 310 and the third stent segment 330 respectively) are subject to the pulsatile motion arising from direct internal contact. These struts in particular should be well secured to the graft material. As illustrated in Figure 6, the stitches 604 on the upper most first stent segment 310 are positioned along the entire zigzag arrangement of struts. The upper and lower apexes of the third stent segment may be stitched utilizing a similar configuration. It is difficult to manipulate the suture thread precisely around the struts that are located some distance away from an open end, accordingly, various other simpler stitches may be utilized on these struts, or no stitches may be utilized in these areas.

As illustrated in Figure 6, each of the struts in the first stent segment 310 is secured to the graft material 600 which has been cut to match the shape of the stent segment 310. The blanket stitching 604 completely encircles the strut and bites into the graft material 600. Preferably, the stitch 604 encircles the strut at approximately five equally spaced locations. Each of the struts on each end of the third stent segment 330 is attached to the graft material, which has been cut to make the shape of the stent segment 330, in the same manner as the first stent segment 310.

A significant portion of the graft will not rest directly against vascular tissue. This portion of the graft will be within the dilated aneurysm itself. Therefore, this portion of the graft will not experience any significant pulsatile motion. For this reason, it is not necessary to secure the stent segments to the graft material as aggressively as the stent structure described above. Therefore, only point stitches 606 are necessary for securing these stents.

It is important to note that a wide variety of sutures are available. It is equally important to note that there are a number of alternative means for attaching the graft material to the stent, including welding, gluing and chemical bonding. If sutures are utilized, any suitable, non-biodegradable or non-biodegradable suture may be utilized. The suture may also be constructed from a degradable material that allows for an acute connection, but also allows for removal of a component if desired after the material degration.

In accordance with another exemplary embodiment, additional material may be added to the grafts to prevent or substantially eliminate graft wear and fiber separation. The additional material is preferably added to the section of graft in areas of expected interaction with the stent structure, for example, where the graft is attached to the stent material. The additional material may be utilized in connection with any of the stent structures described herein, including the trunk and bifurcated sections of the anchoring and sealing component of the repair device and/or the endovascular graft. Essentially, the additional material may be utilized with any medical implant utilizing a woven graft as part of the structure. The added material may comprise any suitable biocompatible material that does not interfere with the operation of the device or with the delivery of the device. The additional material may also include or act as depots for the delivery of therapeutic agents. In the exemplary embodiment described herein, the additional material comprises an epoxy; however, as is discussed subsequently, any number of materials may be utilized.

As described herein, many aneurismal repair devices or simply stent-grafts utilize a woven graft material in combination with a support structure or lattice. The points of contact between the graft material and the support structure most likely to be affected by wear are typically the points where the graft material is affixed or secured to the support structure by sutures or other suitable attachment devices as described herein, and/or around sharp edges or corners of the support structure. Fiber wear and fiber separation within the graft material of the components comprising the aneurismal repair devices may be a potential problem. The pulsatile movement of the artery in which the repair device is positioned, for example, the aorta, causes the stents to rub against the graft material thereby potentially resulting in holes that may cause endoleaks. More specifically, this pulsatile movement will cause the apex of the support structure to move the most relative to the remaining portions of the stent structure. Another potential problem caused by the interaction of the stent structures and the graft material is the separation of the graft fibers. This is caused when part of the stent structure is pressed into the graft material and forces adjacent fibers to separate. This condition may be triggered during manufacturing while handling and loading the device, or in vivo in cases of extreme bending or movement.

Numerous modifications to the type of weave used in a graft or the design of the stent structures have been made over the years to prevent graft wear and separation. Many of these methods are complicated and expensive to incorporate. The present invention is directed to adding an epoxy material to the graft material in areas of expected interaction with the stent structure. The addition of the epoxy should preferably prevent the fibers from separating and also improve wear resistance in two ways. Initially, the epoxy will provide additional material in between the graft fibers, which will delay the wear process and if a graft fiber eventually does break due to wear, the break will be isolated from the rest of the graft material by the epoxy. This will prevent propagation of the hole and weakening of the graft adjacent to the tear. The epoxy may also maintain the integrity of the graft material, in the unlikely event, the graft material within the epoxy region is substantially worn away.

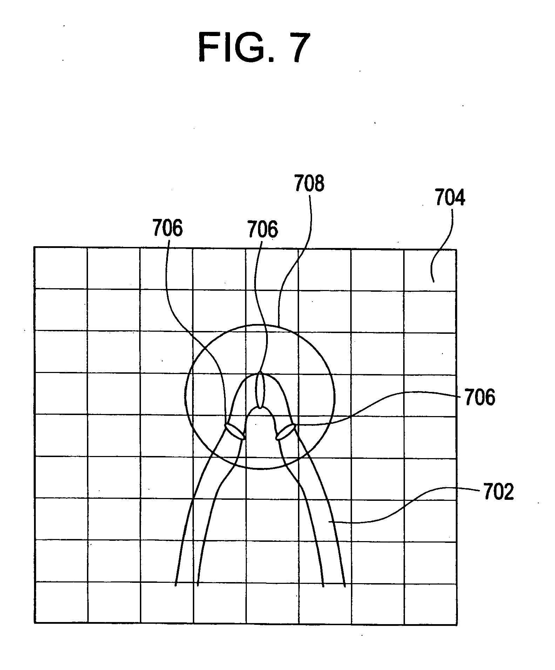

Figure 7 illustrates a portion of a stent structure 702 attached to graft material 704 utilizing sutures 706. As described above, any suitable attachment device may be utilized. The area indicated by the circle 708 represents where an epoxy has been incorporated into and/or affixed to the graft material 704 to increases the durability thereof. The epoxy coating may be applied to the inside of the graft material, to the outside of the graft material and/or soaked through the graft material. Adding epoxy to selected areas prevents the fibers in those areas from separating and localizes any fractures in the fibers. An epoxy or polyepoxide is a thermosetting epoxide polymer that cures when mixed with a hardener. The epoxy may be applied, as described above, to the particular area utilizing any number of well-known techniques. Exemplary epoxies include methyl methacrylate and cyanoacrylate. Methyl methacrylate is a chemical compound typically known as the monomer for the production of polymethyl methacrolate and cyanoacrylate is the generic name for substances such as ethyl-2-cyanoacrylate. It is important to note that other means for selectively reinforcing areas of graft material include adding additional stitching and melting or fusing of the graft material itself. In addition, the graft fibers may be metalized utilizing various techniques such as sputtering or electrodeposition, or thin film fibers may be added to the weave, or the fibers may be coated with a thin metallic film.

In addition to preventing wear, epoxy or other substances may be utilized to selectively increase the stiffness in different areas of the graft. This may be done in addition to high wear areas or just as an alternative thereto. For example, the epoxy or other suitable material or modification may be implemented in any area of the graft material in a pattern that forces the graft material to bend in a predictable manner. This addition may be utilized for any number of purposes including to force the graft material to fold in a predictable manner when loaded into a different system, to focus bending into predictable areas of the graft in vivo and/or to prevent "train-wrecking" of the prosthesis during loading to or delivery from the catheter. Train wrecking is when the prosthesis collapses on itself under compression. In addition, epoxy or other substances may be utilized on just grafts for the reasons described herein.

In accordance with another exemplary embodiment, the epoxy may be applied to the sutures that connect or secure the stent structures to the graft material. Many times adjacent sutures will be created from one continuous run of the suture line. This eliminates the extra knots needed to start and stop a suture and therefore reduces the loaded profile of the prosthesis. In these cases the integrity of many sutures can be compromised by a break at any point along the length of the suture line. If epoxy has been added along the length of the suture/graft interaction and the suture should happen to break, the epoxy would prevent that breakage from affecting the remaining length of suture before the next knot or fixation point. As long as the epoxy is used in close proximity to the stent structures it will not affect the expansion properties or conformability of the graft material.

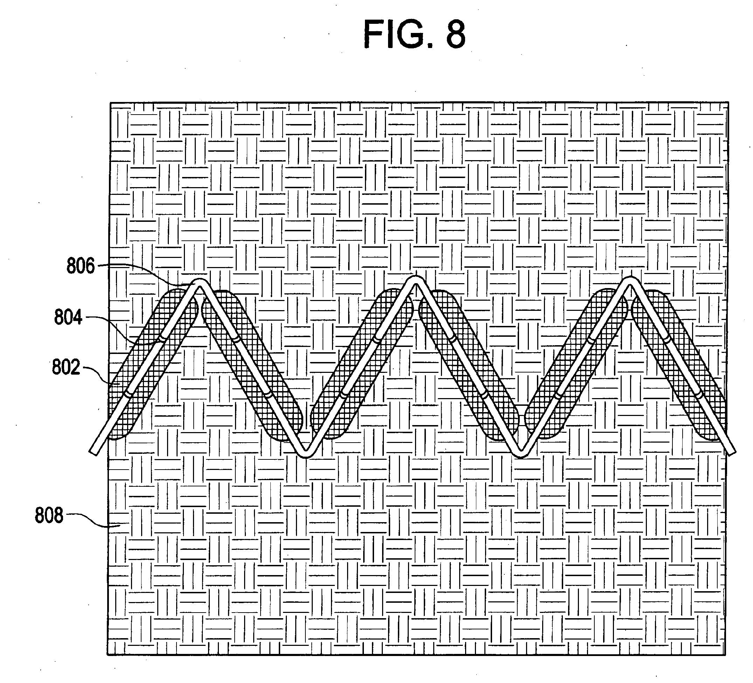

Figure 8 illustrates the above-described exemplary embodiment, wherein an epoxy material 802 is added to or affixed proximate the sutures 804 that secure the stent structure 806 to the graft material 808.

In each of the above-described exemplary embodiments, the epoxy or otherwise modified sections facilitates the distribution of stresses and provides a transition from stiff stent or support structure to supple graft.

Although shown and described is what is believed to be the most practical and preferred embodiments, it is apparent that departures from specific designs and methods described and shown will suggest themselves to those skilled in the art and may be used without departing from the scope of the invention. The present invention is not restricted to the particular constructions described and illustrated, but should be constructed to cohere with all modifications that may fall within the scope for the appended claims.

## Claims

1. An aneurysm repair system comprising:
a graft structure (704, 808); and **characterised in that** the aneurysm repair system further comprises:
an epoxy (708, 802) affixed to predetermined areas of the graft structure (704, 808) to enhance durability and/or stiffness of said predetermined areas.

2. An aneurysm repair system according to claim 1, further comprising:
at least one stent segment (702, 806);
wherein the graft structure (704, 808) is affixed, via attachment elements, to the at least one stent segment (702, 806) to form an endoprosthesis; and
the epoxy (708, 802) affixed to the graft structure (704, 808) proximate to an area of fixed contact between the at least one stent segment (702, 806) and the graft structure (704, 808) to enhance durability.

3. An aneurysm repair system according to claim 1, further comprising:
at least one stent segment (702, 806);
wherein the graft structure (704, 808) is affixed, via attachment elements, to the at least one stent segment (702, 806) to form an endoprosthesis; and
the epoxy (708, 802) is affixed to predetermined areas of the graft material (704, 808) to enhance stiffness.

4. The aneurysm repair system according to Claim 2 or 3, wherein the at least one stent segment (702, 806) comprises a superelastic material.

5. The aneurysm repair system according to claim 4, wherein the superelastic material comprise a nickel-titanium alloy.

6. The aneurysm repair system according to claim 2 or 3, wherein the attachment elements comprise sutures (706, 804).

7. The aneurysm repair system according to any one of the preceding claims, wherein the epoxy (708, 802) comprises cyanoacrylate.

8. The aneurysm repair system according to claim 2 or 3, wherein the graft structure (704, 808) comprises a polyester.

9. An aneurysm repair system according to claim 2, wherein the epoxy (708, 802) is affixed in areas of expected interaction between the graft structure (704, 808) and the at least one stent segment (702, 806).

10. An aneurysm repair system according to claim 1 or 3, wherein the epoxy (708, 802) is affixed in a pattern to enhance the stiffness such that the graft structure (704, 808) folds in a predictable manner or to focus bending into predictable areas of the graft structure (704, 808).

## Patentansprüche

1. Aneurysmareparatursystem, welches eine Implantatstruktur (704, 808) umfasst; und **dadurch gekennzeichnet, dass** das Aneurysmareparatursystem ferner Folgendes umfasst:
ein Epoxid (708, 802), welches an vorbestimmten Bereichen der Implantatstruktur (704, 808) angebracht ist, um eine Haltbarkeit und/oder eine Steifigkeit der vorbestimmten Bereiche zu verbessern.

2. Aneurysmareparatursystem gemäß Anspruch 1, welches weiter Folgendes umfasst:
zumindest ein Stentsegment (702, 806);
wobei die Implantatstruktur (704, 808) mittels Befestigungselementen an dem zumindest einen Stentsegment (702, 806) angebracht ist, um eine Endoprothese zu bilden; und
wobei das Epoxid (708, 802) an der Implantatstruktur (704, 808) in der Nähe eines Bereichs eines festen Kontaktes zwischen dem zumindest einen Stentsegment (702, 806) und der Implantatstruktur (704, 808) angebracht ist, um die Haltbarkeit zu verbessern.

3. Aneurysmareparatursystem gemäß Anspruch 1, welches weiter Folgendes umfasst:
zumindest ein Stentsegment (702, 806);
wobei die Implantatstruktur (704, 808) mittels Befestigungselementen an dem zumindest einen Stentsegment (702, 806) angebracht ist, um eine Endoprothese zu bilden; und
wobei das Epoxid (708, 802) an vorbestimmten Bereichen des Implantatmaterials (704, 808) angebracht ist, um die Steifigkeit zu verbessern.

4. Aneurysmareparatursystem gemäß Anspruch 2 oder 3, wobei das zumindest eine Stentsegment (702, 806) ein überelastisches Material umfasst.

5. Aneurysmareparatursystem gemäß Anspruch 4, wobei das überelastische Material eine Nickel-Titan-Legierung umfasst.

6. Aneurysmareparatursystem gemäß Anspruch 2 oder 3, wobei die Befestigungselemente Nahtmaterialien (706, 804) umfassen.

7. Aneurysmareparatursystem gemäß einem der vorhergehenden Ansprüche, wobei das Epoxid (708, 802) ein Cyanoacrylat umfasst.

8. Aneurysmareparatursystem gemäß Anspruch 2 oder 3, wobei die Implantatstruktur (704, 808) ein Polyester umfasst.

9. Aneurysmareparatursystem gemäß Anspruch 2, wobei das Epoxid (708, 802) in Bereichen einer erwarteten Wechselwirkung zwischen der Implantatstruktur (704, 808) und dem zumindest einen Stentsegment (702, 806) angebracht ist.

10. Aneurysmareparatursystem gemäß Anspruch 1 oder 3, wobei das Epoxid (708, 802) in einem Muster angebracht ist, um die Steifigkeit zu verbessern, so dass die Implantatstruktur (704, 808) auf eine vorhersagbare Weise gefaltet wird oder um ein Biegen auf vorhersagbare Bereiche der Implantatstruktur (704, 808) zu fokussieren.

## Revendications

1. Système de réparation d'un anévrisme comprenant :
une structure de greffon (704, 808) ;
et **caractérisé en ce que** le système de réparation d'anévrisme comprend en outre :
une résine époxy (708, 802) fixée sur des zones prédéterminées de la structure de greffon (704, 808) destinée à améliorer la longévité et / ou la rigidité desdites zones prédéterminées.

2. Système de réparation d'un anévrisme selon la revendication 1, comprenant en outre :
au moins un segment d'endoprothèse (702, 806) ;
dans lequel la structure de greffon (704, 808) est fixée, par l'intermédiaire d'éléments de fixation, sur le ou les segments d'endoprothèse (702, 806) de façon à former une endoprothèse ; et
la résine époxy (708, 802) est fixée sur la structure de greffon (704, 808) à proximité d'une zone de contact fixée entre le ou les segments d'endoprothèse (702, 806) et la structure de greffon (704, 808) de façon à améliorer la longévité.

3. Système de réparation d'un anévrisme selon la revendication 1, comprenant en outre :
au moins un segment d'endoprothèse (702, 806) ;
dans lequel la structure de greffon (704, 808) est fixée, par l'intermédiaire d'éléments de fixation, sur le ou les segments d'endoprothèse (702, 806) de façon à former une endoprothèse ; et
la résine époxy (708, 802) est fixée sur des zones prédéterminées du matériau de greffon (704, 808) de façon à améliorer la rigidité.

4. Système de réparation d'un anévrisme selon la revendication 2 ou la revendication 3, dans lequel le ou les segments d'endoprothèse (702, 806) comprennent un matériau superélastique.

5. Système de réparation d'un anévrisme selon la revendication 4, dans lequel le matériau superélastique comprend un alliage de nickel - titane.

6. Système de réparation d'un anévrisme selon la revendication 2 ou la revendication 3, dans lequel les éléments de fixation comprennent des sutures (706, 804).

7. Système de réparation d'un anévrisme selon l'une quelconque des revendications précédentes, dans lequel la résine époxy (708, 802) comprend un cyanoacrylate.

8. Système de réparation d'un anévrisme selon la revendication 2 ou la revendication 3, dans lequel la structure de greffon (704, 808) comprend un polyester.

9. Système de réparation d'un anévrisme selon la revendication 2, dans lequel la résine époxy (708, 802) est fixée dans des zones d'interaction prévues entre la structure de greffon (704, 808) et le ou les segments d'endoprothèse (702, 806).

10. Système de réparation d'un anévrisme selon la revendication 1 ou la revendication 3, dans lequel la résine époxy (708, 802) est fixée en un motif de façon à augmenter la rigidité de telle sorte que la structure de greffon (704, 808) se plie d'une façon prévisible, ou à concentrer la flexion dans des zones prévisibles de la structure de greffon (704, 808).
